# EUROPEAN PATENT SPECIFICATION

(11) **EP 2 591 822 B1**
(45) Date of publication and mention of the grant of the patent: **18.11.2015**
(21) Application number: 12195358.2
(22) Date of filing: 01.04.2005
(51) Int. Cl.: A61P 25/00, A61P 25/28, A61P 37/00, A61P 17/06, A61K 39/395, A61K 39/42, C07K 16/06, C07K 16/10

(54) **Treatment of diseases**
Behandlung von Krankheiten
Traitement de maladies

(30) Priority: 05.04.2004 GB 0407710; 17.05.2004 GB 0410966
(43) Date of publication of application: 15.05.2013
(62) Divisional of application: 09178431.4
(73) Proprietor: Aimsco Limited, East Sussex BN21 4RL (GB)
(72) Inventor: Youl, Bryan, London NW3 2QG (GB)
(74) Representative: Zvesper, Thomas

(56) References cited:
- WO-A-03/004049
- WO-A-03/064472
- ENGELEN VAN B G M ET AL: "IMPROVED VISION AFTER INTRAVENOUS IMMUNOGLOBULIN IN STABLE DEMYELINATING OPTIC NEURITIS", ANNALS OF NEUROLOGY, BOSTON, US, vol. 32, no. 6, December 1992 (1992-12), pages 834-835, XP000995657, ISSN: 0364-5134
- ROED H G ET AL: "A double-blind, randomized trial of IV immunoglobulin treatment in acute optic neuritis", NEUROLOGY, vol. 64, no. 5, 8 March 2005 (2005-03-08), pages 804-810, XP002357191, ISSN: 0028-3878
- DATABASE EMBASE [Online] ELSEVIER SCIENCE PUBLISHERS, AMSTERDAM, NL; 1997, DALAKAS M C: "Intravenous immune globulin therapy for neurologic diseases", XP002357192, Database accession no. EMB-1997129400 & ANNALS OF INTERNAL MEDICINE 1997 UNITED STATES, vol. 126, no. 9, 1997, pages 721-730, ISSN: 0003-4819
- DODEL R ET AL: "HUMAN ANTIBODIES AGAINST AMYLOID BETA PEPTIDE: A POTENTIAL TREATMENT FOR ALZHEIMER'S DISEASE", ANNALS OF NEUROLOGY, JOHN WILEY AND SONS, BOSTON, US, vol. 52, no. 2, 1 August 2002 (2002-08-01) , pages 253-256, XP009026281, ISSN: 0364-5134, DOI: 10.1002/ANA.10253

## Description

### FIELD OF THE INVENTION

The present invention relates to compositions for use in the treatment of neural disorders. In particular but not exclusively, the invention relates to compositions for restoring or improving neural transmission in damaged nerve cells. Certain aspects of the invention relate to compositions for use in the treatment of non-demyelinating neural disorders.

### BACKGROUND OF THE INVENTION

PCT publications WO03/004049 and WO03/064472 describe therapeutic agents and treatments which are based on a serum composition with many surprising beneficial effects. In particular, the reader is referred to them for an understanding of how the therapeutic agent can be prepared, and for the indications which can be treated.

Typically a goat is immunised with HIV-3B viral lysate raised in H9 cells. The resulting serum is believed to be active against HIV, and multiple sclerosis. The reader is further referred in particular to the section on pages 3 and 4 of WO03/004049 headed 'Example of Production of Goat Serum' for further details of the production of serum. The use of HIV-3B viral lysate as an immunogen is not believed to be essential for the production of active serum; it is believed that a medium which has been used for growth of a viral culture, or which is suitable for such growth, may also produce a suitable response when used as an immunogen, The supernate of a cell culture growth medium such as PBMC or the cancer immortal cell line as used to grow HIV-3B are given as an example. The HIV or other virus does not need to be present to produce an effective immunogen to create the composition. Other suitable immunogens are recited on pages 12 and 13 of WO03/064472.

The serum is believed to be effective against HIV and against multiple sclerosis. An important component of the activity of the serum is suggested in WO03/064472 as being anti-HLA or anti-FAS activity. Such antibody components would be expected to be relatively slow acting.

The same publication also suggests that the serum may be used to treat traumatic axonal or nerve damage, and that the serum may also include some neural growth factor (HGF) activity, and may function in remyelinating demyelinated traumaticaily damaged nerves. Again, such activity would be expected to be relatively slow acting.

The present applicants have now surprisingly determined that the serum composition has an additional rapid effect on deteriorated nerves, with preliminary observations suggesting that a relatively rapid restoration of transmission of nerve impulses may occur. It is thought that this rapid effect may apply to both demyelinated and non-demyelinated deteriorated nerves.

The detection of this rapid effect on nerve transmission suggests that the serum must contain some active component other than NGF activity, which is relatively fast acting. This realisation leads to important new insights regarding the nature of neural disorders which may be treated with the serum. In particular, the inventors have now realised that the serum may be effectively used for the treatment of neural disorders which are non-demyelinating, and for the treatment of non-traumatic demyelinating disorders. These insights have been based on the newly-identified rapid effect on nerve transmission, and would not have been expected based on the previously-identified slow NGF-like activity imputed to the serum.

Further, the inventors believe that the serum composition may be useful in the treatment of certain autoimmune diseases. There are a number of autoimmune or immune-mediated diseases found in humans. Current therapies for such diseases generally involve either the administration of immune suppressants, or the administration of steroids.

Some of the main autoimmune diseases are as follows.

Systemic lupus erythematosus is a chronic autoimmune disease in which the patient's antibodies attack healthy tissues and organs. The severity may range from mild to life threatening. Lupus may also affect the skin, causing a rash and lesions, usually across the face and upper part of the body.

Psoriasis is a common, chronic skin disorder which is believed to be autoimmune in nature,
Eczema is another common skin disorder, which is believed to have an autoimmune component
Thyroiditis mainly has an autoimmune cause, in which the patient's antibodies attack the thyroid.

Polymyositis is an autoimmune neuromuscular diseases, leading to limb and neck weakness, sometimes associated with muscle pain.

There is a need for an alternative treatment for these disorders.

Dodel *et al.* (2002) discusses human antibodies against amyloid peptide as a potential treatment for Alzheimer's disease.

### SUMMARY OF THE INVENTION

According to a first aspect of the present invention, there is provided a serum composition for use according to claim 1.

The serum composition may be as previously described in WO03/004049 and WO03/064472. The immunogen may comprise HIV, in intact host cells, cell-free extracts, viral lysate, or a mixture thereof. Alternatively, the immunogen may be a medium suitable for growth of a viral culture. Other suitable immunogens are described in WO03/004049 and WO03/064472. An example of preparation of goat serum is given below.

The non-demyelinating disorder which is treated in accordance with the present invention is Alzheimer's disease. This disorder may have an inflammatory component, but is believed to be additionally treatable based on the non-demyeiinating neural aspect of the disorder.

The serum composition is preferably administered in a dosage of between 0.01 and 10 mg/kg to the subject; more preferably between 0.01 and 5 mg/kg, between 0.05 and 2 mg/kg, and most preferably between 0.1 and 1 mg/kg, The precise dosage to be administered may be varied depending on such factors as the age, sex and weight of the patient, the method and formulation of administration, as well as the nature and severity of the disorder to be treated. Other factors such as diet, time of administration, condition of the patient, drug combinations, and reaction sensitivity may be taken into account. An effective treatment regimen may be determined by the clinician responsible for the treatment. One or more administrations may be given, and typically the benefits are observed after a series of at least three, five, or more administrations.

The serum composition may be administered by any effective route, preferably by subcutaneous injection, although alternative routes which may be used include intramuscular or intralesional injection, oral, aerosol, parenteral, or topical.

The serum is preferably administered as a liquid formulation, although other formulations may be used. For example, the serum may be mixed with suitable pharmaceutically acceptable carriers, and may be formulated as solids (tablets, pills, capsules, granules, etc) in a suitable composition for oral, topical or parenteral administration.

The serum according to the invention may also be used in the preparation of a medicament for treatment of Alzheimer's disease.

Surprisingly, it has been identified that the action of the serum on damaged nerves may be effective on both demyelinated and non-demyelinated nerves.

The serum composition may, but need not, comprise anti-HLA antibody. It is believed that this may play a role in the activity of the serum.

Examples of pharmaceutical compositions include any solid (tablets, pills, capsules, granules, ointments, etc) with suitable composition for oral, topical, or parenteral administration; fluids suitable for injection; or aerosols suitable for administration to a patient. The compositions may include a carrier.

It is believed that at least a component of the serum activity is linked with anti-HLA activity; the activity may reside in the antibody itself or in some other factor associated with the antibody. Preferably the anti-HLA antibody is goat anti-HLA antibody. The antibody may be polyclonal.

### BRIEF DESCRIPTION OF THE DRAWINGS

Figure 1. "Visual Evoked Potentials pre-treatment".
   Pre-treatment Visual evoked potentials, recorded from the occipital cortex in response to standard checkerboard stimuli (OZ-FZ). a) 3 individual runs, b) The same runs, superimposed, c) Mean response.
Figure 2. "Visual Evoked Potentials post-treatment".
   Post-treatment. Using the same recording paradigm as for Figure 1, 30 minutes after sub-cutaneous injection of Aimspro. a) 2 individual runs, b)

The same runs, superimposed. c) Mean response. A reproducible P100 response is now evidence, with a markedly delayed latency of 165ms.

### DETAILED DESCRIPTION OF THE INVENTION

### Example of Production of Goat Serum

A goat was inoculated by intramuscular injection with lysed HIV viral cocktail and formulated with Freund's adjuvant. The virus was previously heat killed at 60°C for 30 minutes. Blood samples were drawn after an appropriate interval, such as two weeks, for initial assessment. In the optimised procedure, the goat is injected every week for four weeks, then at six weeks the animal is bled to obtain the reagent.

Approximately 400 cc of blood is drawn from the goat under sterile technique. The area for needle extraction is shaved and prepared with betadine. An 18-gage needle is used to draw approximately 400 cc of blood from the animal. Of note is that the animal can tolerate approximately 400 cc of blood drawn without the animal suffering any untoward effects. The animal does not have to be sacrificed. The animal can then be re-bled in approximately 10 to 14 days after it replenishes its blood volume.

The presence of potentially useful antibodies was confirmed, having regard to the desired antibody activity. Once the presence of such reagents was confirmed, blood was then taken from the goat at between 4-6 weeks.

The base blood product in order to create the reagent is then centrifuged to create the serum. 300 ml of serum was then filtered to remove large clots and particulate matter. The serum was then treated with supersaturated ammonium sulphate (45% solution to room temperature), to precipitate antibodies and other material. The resulting solution was centrifuged at 5000 rpm for five minutes, after which the supernatant fluid was removed. The precipitated immunoglobulin was resuspended in phosphate-buffered saline (PBS buffer, see Sambrook et al, 'Molecular Cloning: A Laboratory Manual', 1989) sufficient to redissolve the precipitate.

The solution was then dlalysed through a membrane with a molecular weight cut off of 10,000 Daltons. Dialysis was carried out in PBS buffer, changed every four hours over a period of 24 hours. Dialysis was carried out at 4°C.

After 24 hours of dialysis the contents of the dialysis bag were emptied into a sterile beaker. The solution was adjusted such that the mass per unit volume = 10 mg per ml. The dilution was carried out using PBS. The resulting solution was then filtered through a 0.2 micron filter into a sterile container. After filtration, the solution was aliquoted into single dosages of 1ml and stored at - 22°C prior to use. The composition is referred to herein as AIMSPRO serum.

### Neural disorders

Acute optic neuritis is a common manifestation of multiple sclerosis. It presents as an episode of monocular blurring of central vision, with a pronounced effect on colour discrimination. While spontaneous resolution usually follows, successive attacks may result in irreversible and often slowly progressive, visual loss¹. No medication has been available to improve visual function in these chronically affected patients. Here we present evidence of a promising approach to therapy along with electrophysiological indications of a remarkable rapidity of onset.

Six multiple sclerosis patients with stable visual dysfunction due to chronic optic neuropathy (2 males, 4 females, aged from 32 to 42 years, disease duration 8 to 16 years) were treated with a product referred to as Aimspro, which is obtained from purified goat serum as described above and in WO03/004049 and WO03/064472. Administration of the drug was 1ml by sub-cutaneous injection, generally self-administered after the first or second dose. The frequency of administration, adjusted according to response, varied from once, to three times weekly. No patient had received the product previously, but one (Case 2) had been taking interferon beta-1a (Rebif) for nearly a year: this treatment was ceased the day prior to treatment with Aimspro. Recordings were carried out immediately prior to the first Injection, and at approximately one hour, one week and 4 to 7 weeks thereafter. Prior to treatment, all subjects described that their vision had slowly and progressively deteriorated over periods of from 3 to 14 years, and none could recall intervening periods of what may have represented acute optic neuritis.

Corrected distance acuity (Snellen chart) and colour vision (square root of total error score from the Farnsworth-Munsell 100-Hue test²) data, acquired under standardized lighting conditions, are presented (Table). Monocular visual evoked potential (VEP) studies were carried out on each occasion. Perimetry was not performed. Sub-lingual temperature was monitored and showed no significant variability, within subjects, over time. Data from left and right eyes were considered to be independent for analysis and colour vision scores were treated as non-parametric for statistical purposes.

Comparison of pre-treatment and follow-up distance acuities showed no significant change and in only two eyes (Case 2 left eye and Case 5 right eye) was there an improvement of one line or more on the Snellen chart. A repeated measures analysis of variance (ANOVA) test on the colour vision scores, however, yielded F=(2.16, 23.73)=8.52, p=0.001. Within approximately one hour of injection, there was significant improvement in colour vision (p=0,008, Z=-2.667, Wilcoxon signed ranks test). Comparison of pre-treatment and "one week" values showed no significant difference (p=0.055, Z=-1.923) but comparison of pre-treatment and follow up data (at 4 to 7 weeks) showed significant benefit (p=0.003, Z=-2.981). No significant side effects other than local pain and swelling at injection sites over the first two to three weeks, in three patients, were encountered.

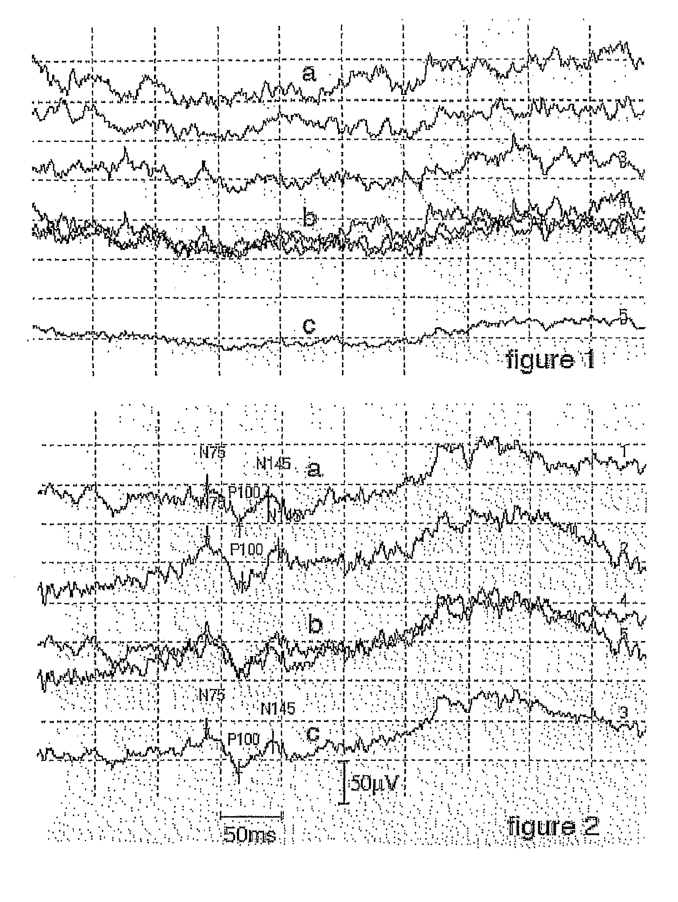

For cases 5 and 6, VEP response latencies lay towards the upper limits of normal. Pre-treatment VEP studies from all but one of the remaining eyes showed delay in the P100 response, consistent with demyelination within visual pathways. In only one instance (Case 4 right eye) was no response obtainable prior to treatment and this was the only eye from the entire series to show a significant change in averaged cortical responses at any time during the observation period. This 42 year old female with secondary progressive multiple sclerosis of spinal onset in 1992, had complained of gradually deteriorating vision since 1998. There had been four periods of 3 to 7 days' duration of resolving blurring of vision between 1993 and 1997, but there had been no more recent episodic visual features in the history. Examination showed bilateral optic atrophy and marked impairment of distance and colour vision. Pre-treatment full field pattern reversal VEP studies at 15:02hrs yielded no reproducible tracings from the right eye (see figure 1). A test dose of Aimspro (0.1ml) was administered subcutaneously at 15:13hrs, followed by an additional 0.9ml at 15:25hrs. A markedly delayed but reproducible P100 response could now be obtained at 15:43hrs, at 171ms (see figure 2). The scalp leads had remained attached throughout the study and test conditions, including body temperature, were monitored. While this neurophysiological finding was consistent with reversal of conduction block in severely demyelinated fibres³, it was not accompanied by a clinically significant improvement in acuity data. The fact that no improvement in P100 latency could be detected from any eye over the study period argues against there having been significant remyelination during this time, further but observations at perhaps 6 months would be needed to assess this adequately.

In summary, non-blinded, uncontrolled observations in 6 patients with slowly progressive visual dysfunction due to optic neuritis, show a significant improvement in colour vision over the course of between 4 and 7 weeks of treatment with a novel medication, Aimspro. Neurophysiological data from one affected eye in a patient with a five year history of marked visual deficit are consistent with an interpretation that the drug administration caused a reversal of axonal conduction block. Moreover, while this phenomenon was shown to have occurred within 30 minutes of treatment, a clinical observation by the author (unpublished observation) on a 38 year old female patient with a "spinal" relapse of relapsing remitting multiple sclerosis, suggests that "unblocking" may be seen within as little as ten minutes. A further clinical observation (unpublished observation) on a patient with 18 years of stable motor deficit following severe Guillain-Barré syndrome suggests that the effect may pertain to the peripheral nervous system as well.

Visual deficit in acute optic neuritis (as gauged by clinical and neurophysiological examination) is thought to reflect axonal conduction block related to local inflammatory demyelinating activity^{4,5,6}, but inflammation seems unlikely to be a persisting factor in chronically affected cases such as the six patients described above. A direct effect of a component of the medication on nerve transmission is, therefore, suspected. Basic neurophysiological techniques are now being harnessed with a view to clarifying the mechanism of action.

Aimspro is a serum product initially intended to provide high titer neutralizing antibodies for use in HIV patients. Characterization of the serum has revealed a high titer of anti-HLA class 2 antibodies which are able to inhibit a variety of mixed lymphocyte reactions (unpublished observations). As increased HLA class 2 expression on brain cells and lymphocytes is recognized to be a major factor in the inflammatory process in multiple sclerosis, it was postulated that the polyclonal serum may be beneficial in multiple sclerosis and similar conditions (for review see Reference 1). Indeed, monoclonal antibodies against HLA class 2 are under development by several companies. However, the rapidity of the clinical responses seen here suggests that other mechanisms may be operating in vivo. A delay in the inactivation of sodium channels, and the blockade of potassium channels have both been shown to improve conduction in experimentally demyelinated axons⁷. Alternatively, a removal of blockade of axonal sodium channels by endogenous substances such as nitric oxide⁸ may explain the rapidity of the drug effect. It is therefore possible that in addition to any effects that the serum may have in influencing immunological events, it may also affect the security of axonal conduction directly.

### Autoimmune disorders

The Aimspro product may also be used for treatment of autoimmune disorders as follows, A 1 ml aliquot of serum, prepared as described, is adjusted to provide a dose of 0.1 mg/kg, and injected subcutaneously to a patient suffering from an autoimmune disease selected from the group comprising lupus, psoriasis, eczema, thyroiditis, and polymyositis.

The product was given to a patient as follows. The male patient experienced psoriasis *de novo* with a first presentation which started on the hands but spread over most of lower legs and arms. The treating physician prescribed Timodine, then Mometasone. By the end of the month, the condition was widespread. Prescribed Polytar emollient and referred to consultant dermatologist who confirmed acute psoriasis, and prescribed Mometasone, Polytar and Exorex. The treatment had little effect, with psoriasis worst on arms and legs. Commenced AIMSPRO product on day 1, 1ml weekly. Day 5, psoriasis started improving. Day 23, exfoliating much improved. Increase in dose to 2 amps weekly. After 2 months, patient much improved, and by 3 months and 18 days, psoriasis now considered resolved, and the patient wished to stop treatment. Thus given 1 amp weekly for 4/52, 2 amps weekly for 12/52; in total 28 amps over 16 weeks. There were no side effects reported.

### References.

1. Compston A, Coles A. Multiple Sclerosis, Lancet 2002; 359;1221-31
2. Farnsworth D. The Farnsworth-Munsell 100-Hue and Dichotomous Tests for Color Vision. J Opt Soc Am, 33, 568 (1943).
3. McDonald WI, Sears TA. The effect of experimental demyelination on conduction in the central nervous system. Brain 1970; 93, 583-598.
4. Hawkins CP, et al. Duration and selectivity of blood-brain barrier breakdown in chronic relapsing experimental allergic encephalomyelitis studied by gadolinium-DTPA and protein markers. Brain 1990; 113, 365-378.
5. Katz D, Taubenberger J, Raine C, McFarlin D, McFarland H. Gadoliniumenhancing lesions on magnetic resonance imaging. Ann Neurol 1990; 28, 243.
6. Youl BD, et al The pathophysiology of acute optic neuritis: an association of gadolinium leakage with clinical and electrophysiological deficits. Brain 1991; 114; 2437-2450.
7. Smith KJ, McDonald WI. The pathophysiology of MS: the mechanisms underlying the production of symptoms and the natural history of disease. Philos Trans R Soc Lond B Biol Sci 1999; 354: 1649-1673.
8. Redford EJ, Kapoor R and Smith KJ. Nitric oxide donors reversibly block axonal conduction: demyelinated axons are especially susceptible. Brain Part 12 (Dec 1997) 2149-57.

### TABLE LEGEND:

MS TYPE SP (Secondary Progressive) RR (Relapsing Remitting)
Dx yrs Years since probable onset of multiple sclerosis
VsDtn yrs Years of progressive visual loss
EYE Right and left eyes are treated independently
P100 ms The P100 VEP positivity latency in milliseconds
VA pre Snellen chart derived visual acuity pre-treatment
VA 1 hour As above, at about 1 hour post treatment
VA 7 days As above, at 7 days
VA FU As above, at follow-up (4-7 weeks)
CV pre Square root of the Farnsworth-Munsell 100-Hue Test
CV 1 hour As above, at about 1 hour post treatment
CV 7 days As above, at 7 days
CV FU As above, at follow-up
NR No response

**Demographic, Psychophysical Neurophysiological Data.**

| MSTYPE | Dx yrs | VsDtn yrs | EYE | P100 ms | VA pre | VA 1 hour | VA 7days | VAFU | CV pre | CV post | CV 7days | CV FU |
|---|---|---|---|---|---|---|---|---|---|---|---|---|
| SP | 16 | 14 | R | 146 | 66-3 | 66-2 | 66 | 66 | 11.83 | 10.77 | 10.77 | 10.2 |
| | | | L | 158 | 69-2 | 69-2 | 66-1 | 66-2 | 15.23 | 11.66 | 9.8 | 9.8 |
| SP | 9 | 3 | R | 152 | 66-1 | 66-1 | 66-1 | 66 | 7.75 | 8.72 | 6.93 | 6.32 |
| | | | L | 161 | 324-1 | 312-1 | 312-1 | 312-1 | 21.82 | 19.8 | 19.08 | 12.33 |
| SP | 8 | 6 | R | 173 | 618 | 612 | 618+1 | 618 | 21.26 | 17.2 | 16.37 | 13.42 |
| | | | L | 207 | 618-1 | 618+1 | 618 | 618 | 19.6 | 18.97 | 19.18 | 12.96 |
| SP | 12 | 5 | R | NR | 118-1 | 19-1 | 118 | 118+1 | 30.59 | 27.86 | 33.29 | 29.33 |
| | | | L | 161 | 318-1 | 318 | 318 | 318+1 | 27.42 | 25.69 | 28.21 | 27.78 |
| SP | 16 | 14 | R | 112 | 336 | 324 | 336+2 | 318-1 | 14.97 | 13.56 | 11.66 | 12.96 |
| | | | L | 115 | 618 | 618 | 618 | 68+1 | 14.28 | 13.11 | 11.83 | 10.95 |
| RR | 14 | 4 | R | 114 | 66 | 66 | 66 | 66 | 7.21 | 6.63 | 7.75 | 5.29 |
| | | | L | 114 | 66 | 66 | 66 | 66 | 7.75 | 7.75 | 7.21 | 6.02 |

## Claims

1. A serum composition obtained from a goat following challenge with an immunogen for use in the treatment of Alzheimer's disease, wherein the immunogen is HIV.

2. The serum composition for the use of claim 1, wherein the composition is to be administered in a dosage of between 0.01 and 10 mg/kg to a subject.

3. The serum composition for the use of claim 1 or claim 2, wherein the composition is to be administered by a delivery route selected from subcutaneous injection, intramuscular injection, intralesional injection, oral, aerosol, parenteral or topical delivery.

4. The serum composition for the use of any one of the preceding claims, wherein the immunogen is HIV in intact host cells, cell-free extracts, viral lysate or a mixture thereof.

5. The serum composition for the use of any one of the preceding claims, wherein the immunogen is HIV -3B viral lysate.

## Patentansprüche

1. Serumzusammensetzung, die aus einer Ziege nach der Herausforderung mit einem Immunogen erhalten wird, zur Verwendung bei der Behandlung der Alzheimer-Krankheit, wobei das Immunogen HIV ist.

2. Serumzusammensetzung zur Verwendung nach Anspruch 1, wobei die Zusammensetzung in einer Dosierung von 0,01 bis 10 mg/kg an einen Patienten zu verabreichen ist.

3. Serumzusammensetzung zur Verwendung nach Anspruch 1 oder 2, wobei die Zusammensetzung über einen Zufuhrweg zu verabreichen ist, der aus Folgendem ausgewählt wird:
subcutane Injektion, intramuskuläre Injektion,
intraläsionale Injektion, oral, aerosol, parenteral oder topisch.

4. Serumzusammensetzung zur Verwendung nach einem der vorhergehenden Ansprüche, wobei das Immunogen HIV in intakten Wirtszellen, zellfreien Extrakten, Viruslysat oder einer Mischung davon ist.

5. Serumzusammensetzung zur Verwendung nach einem der vorhergehenden Ansprüche, wobei das Immunogen HIV -3B Viruslysat ist.

## Revendications

1. Composition sérique obtenue à partir d'une chèvre après provocation avec un immunogène, destinée à une utilisation dans le traitement de la maladie d'Alzheimer, l'immunogène étant le VIH.

2. Composition sérique destinée à l'utilisation selon la revendication 1, la composition étant destinée à une administration à un sujet à une dose de 0,01 mg/kg à 10 mg/kg.

3. Composition sérique destinée à l'utilisation selon la revendication 1 ou la revendication 2, la composition étant destinée à une administration par une voie d'administration sélectionnée parmi une administration par injection sous-cutanée, par injection intramusculaire, par injection intralésionnelle, par voie orale, par aérosol, par voie parentérale ou par voie topique.

4. Composition sérique destinée à l'utilisation selon l'une quelconque des revendications précédentes, l'immunogène étant le VIH dans des cellules hôtes intactes, des extraits acellulaires, un lysat viral ou un mélange de ceux-ci.

5. Composition sérique destinée à l'utilisation selon l'une quelconque des revendications précédentes, l'immunogène étant un lysat viral de type VIH-3B.
